(19) Europäisches Patentamt
European Patent Office
Office européen des brevets

(11) Publication number: **0 257 454**
**A1**

# (12) EUROPEAN PATENT APPLICATION

(21) Application number: **87111680.2**

(22) Date of filing: **12.08.87**

(51) Int. Cl.4: **A 61 K 9/10**, A 61 K 47/00, A 61 K 31/415

(30) Priority: **18.08.86 JP 193595/86**

(43) Date of publication of application: **02.03.88 Bulletin 88/9**

(84) Designated Contracting States: **AT BE CH DE ES FR GB IT LI NL SE**

(71) Applicant: **MORISHITA PHARMACEUTICAL CO. LTD., No. 4-29, Doshu-cho Higashi-ku, Osaka-shi Osaka-fu (JP)**

(72) Inventor: **Mito, Yasuo, No. 1823-1, Oshinohara Yasu-cho, Yasu-gun Shiga-ken (JP)**
Inventor: **Tagami, Mitsuko, No. 1823-1, Oshinohara Yasu-cho, Yasu-gun Shiga-ken (JP)**
Inventor: **Kishida, Yukiyo, No. 1823-1, Oshinohara Yasu-cho, Yasu-gun Shiga-ken (JP)**
Inventor: **Fujii, Mitsuharu, No. 1823-1, Oshinohara Yasu-cho, Yasu-gun Shiga-ken (JP)**
Inventor: **Shimazu, Hajime, No. 1600-138, Oaza-Minamizakura Yasu-cho, Yasu-gun Shiga-ken (JP)**
Inventor: **Shiraishi, Sumihiro, No. 200-24, Hashimoto-cho Seta, Otsu-shi Shiga-ken (JP)**
Inventor: **Taziri, Fumio, No. 1691-31, Mabuchi-cho, Omi-Hachiman-shi Shiga-ken (JP)**
Inventor: **Yaginuma, Hideya, No. 2030-89, Mikumo Kousei-cho, Koka-gun Shiga-ken (JP)**

(74) Representative: **Vossius & Partner, Siebertstrasse 4 P.O. Box 86 07 67, D-8000 München 86 (DE)**

## (54) Oil-in-water type fat emulsion of 1-[2-(2,4-dichlorophenyl)-3-methyl-1-pentenyl]-1H-imidazole.

(57) An oil-in-water type fat emulsion comprising (a) 0.01 to 20% (w/v) of 1-[2-(2,4-dichlorophenyl)-3-methyl-1-pentenyl]-1H-imidazole,

(b) 5 to 50% (w/v) of at least one fat emulsion base selected from the group consisting of vegetable oils, triglycerides of a fatty acid having 8 to 12 carbon atoms, and di- and mono-glycerides of a fatty acid having 6 to 18 carbon atoms,

(c) 0.05 to 5% (w/v) of at least one emulsifying agent selected from the group consisting of a phospholipid having a content of phosphatidylcholine of 85% (w/w) or more and a nonionic surfactant, and

(d) remainder of water, and optionally containing further an isotonic agent and/or a stabilizing agent, said fat emulsion being useful for the treatment of various mycotic diseases effectively and safely.



ACTORUM AG

VOSSIUS & PARTNER
PATENTANWÄLTE
SIEBERTSTR. 4
8000 MÜNCHEN 86

0 257 454

u. Z.: W 759 EP
Case: 615191
Morishita Pharmaceutical Co.,Ltd.
Osaka, Japan

August 12, 1987

# OIL-IN-WATER TYPE FAT EMULSION OF 1-[2-(2,4-DICHLOROPHENYL)-3-METHYL-1-PENTENYL]-1H-IMIDAZOLE

This invention relates to a new pharmaceutical composition of an imidazole compound having excellent antifungal activity, more particularly, to an oil-in-water type fat emulsion containing 1-[2-(2,4-dichlorophenyl)-3-methyl-1-pentenyl]-1H-imidazole of the formula:

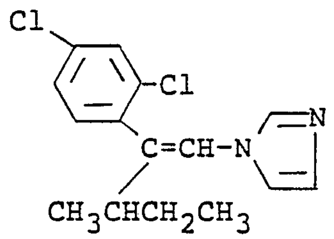

[I] .

which is useful for the treatment of various mycotic diseases.

## Prior Art

The compound of the formula [I] is disclosed in Japanese Patent Second Publication (Kokoku) No. 30673/1985, in which it is disclosed that the compound has a wide range of antifungal spectrum and is useful as an antifungal agent for the treatment of deep mycotic infections by oral, topical, intraperitoneal, parenteral, or rectal administration. Particular pharmaceutical preparations, however, are not disclosed.

It is also known that various drugs can be prepared in various types of pharmaceutical preparations suitable for oral or parenteral administration. Among them, it is known

that fat emulsions are suitable for some medicaments. For instance, Japanese Patent First Publication (Kokai) No. 44809/1986 (= U.S. Patent 4,647,586) discloses a fat emulsion containing 4-biphenylylacetic acid compounds having antiinflammatory, analgesic or antipyretic activities, which comprises an active ingredient, a fat emulsion base, an emulsifying agent, and water, and optionally an isotonic agent and a stabilizing agent.

Japanese Patent First Publication (Kokai) No. 149524/1985 discloses a fat emulsion of prostaglandin, which comprises prostaglandin, oil components, phospholipids, water and optionally an emulsifying agent, a stabilizing agent, etc., which is characteristic in use of a phospholipids containing no phosphatidylethanolamine.

U.S. Patent 4,168,308 and 4,073,943 disclose a composition for enhancing parenteral administration of water-insoluble pharmacologically active agents which comprises an oil-in-water emulsion containing a lipoid as a hydrophobic phase dispersed in a hydrophilic phase and an active ingredient dissolved in said lipoid.

U.S. Patent 3,169,094 discloses intravenously injectable fat emulsions of vegetable oils and phosphatides free of undesirable side effects which are prepared by emulsifying an aqueous mixture containing soybean oil and egg phosphatides, and subsequently homogenizing said mixture to produce an aqueous emulsion.

The present inventors have studied the activities of the active compound [I] and of pharmaceutical preparation thereof, and have found that the compound shows less effect when administered orally but shows excellent antifungal activity when administered by injection, and further that when the water-insoluble compound [I] is prepared in the form of an injectable aqueous solution by converting it into a salt with an acid (e.g. hydrochloride) to make water-soluble, followed by dissolving the salt in water, or by dissolving the compound [I] in water with a solubilizer such as surfactants, the compound [I] shows undesirably significant irritation to the blood vessel.

Summary Description of the Invention

The present inventors have further intensively studied - suitable forms of a pharmaceutical preparation of the compound [I] and have found that it can favorably be used in the form of an oil-in-water type fat emulsion.

An object of the invention, therefore, is to provide an oil-in-water type fat emulsion of the active compound [I] which can exhibit the activities of the compound [I] with high safety. This and other objects and advantages of the invention will be apparent to persons skilled in the art from the following description.

Detailed Description of the Invention

The pharmaceutical composition of this invention is

an oil-in-water type fat emulsion, which comprises

(a) 0.01 to 20 % (w/v) of the active compound [I],

(b) 5 to 50 % (w/v) of at least one fat emulsion base selected from the group consisting of vegetable oils, triglycerides of a fatty acid having 8 to 12 carbon atoms, and di- and mono-glycerides of a fatty acid having 6 to 18 carbon atoms,

(c) 0.05 to 5 % (w/v) of at least one emulsifying agent selected from the group consisting of a phospholipid having a content of phosphatidylcholine of 85 % (w/w) or more and a nonionic surfactant, and

(d) remainder of water,

which may optionally contain further an isotonic agent and/or a stabilizing agent.

The oil-in-water type fat emulsion of the invention (hereinafter, referred to as merely "fat emulsion") can be prepared by dissolving the active compound [I] in particles of a conventional fat emulsion base, and then dispersing the active compound-containing particles into water with an emulsifying agent.

The fat emulsion base used in the preparation of the present fat emulsion includes conventional pharmaceutically acceptable fats and oils, such as vegetable oils (e.g. soybean oil, cotton seed oil, rape oil, safflower oil, etc.), triglycerides of a medium fatty acid having 8 to 12 carbon atoms (e.g. caprylic acid, capric acid, lauric acid,

etc.), di- or mono-glycerides of a fatty acid having 6 to 18 carbon atoms (e.g. caproic acid, capric acid, myristic acid, palmitic acid, linolic acid, stearic acid, etc.), and the like, which can be used alone or in combination of two or more thereof. Among these, preferred one is soybean oil and a commercially available synthetic triglyceride, for example, Panacate® 810 (a mixture of triglycerides of medium fatty acids, manufactured by Nippon Oil and Fats Co., Ltd.). The amount of these fat emulsion base is not critical but is variable depending on the kinds and amounts of the compound [I] and/or other components, and is preferably in the range of 5 to 50 % (w/v), more preferably 5 to 20 % (w/v).

The emulsifying agent is used for dispersing stably the fat emulsion base containing the compound [I] into water and includes phospholipids having a phosphatidylcholine content of 85 % (w/w) or more and nonionic surfactants, which may be used alone or in combination of two or more thereof.

The phospholipids include purified soybean phospholipids, phosphatidylcholine, and the like. Although it is known that a purified egg yolk phospholipid is usually used as an emulsifying agent in the preparation of fat emulsions, it has now been found that it has a low phosphatidylcholine content such as about 75 % (w/v), and hence, it does not show sufficient emulsifying effect for dispersing stably the

fat emulsion base containing the compound [I] into water.

The nonionic surfactant includes, for example, polyoxyalkylene copolymers (e.g. polyoxyethylene-polyoxypropylene copolymer having an average molecular weight of 1,000 to 20,000, etc.), hydrogenated castor oil polyoxyalkylene derivatives (e.g. hydrogenated castor oil polyoxyethylene-(40) ether, hydrogenated castor oil polyoxyethylene-(20) ether, etc.), sorbitan fatty acid ester ethylene oxide adducts (e.g. tween 80, etc.), and the like.

The above phospholipids and nonionic surfactants may be used alone or in combination of two or more thereof and are preferably used in an amount of 0.05 to 5 % (w/v), more preferably 0.5 to 3 % (w/v).

The fat emulsion of this invention may optionally be incorporated by additional additives, such as an isotonic agent and a stabilizing agent.

The isotonic agent (i.e. an agent for making the composition isotonic with body fluids) includes, for example, sugar alcohols (e.g. glycerin, sorbitol, xylitol, etc.), mono- or di-saccharides (e.g. glucose, fructose, maltose, etc.), amino acids (e.g. L-alanine, L-valine, glycine, etc.), and the like, which may be used alone or in combination of two or more thereof. The isotonic agent is usually used in an amount of 1 to 5 % (w/v).

The stabilizing agent includes cholesterol and tocopherol. Cholesterol is usually used in an amount of not

more than 1.2 % (w/v), preferably 0.2 to 0.4 % (w/v), and tocopherol is usually used in an amount of not more than 2.5 % (w/v), preferably 0.2 to 0.8 % (w/v). Besides, albumin can also be used as a stabilizing agent in an amount of 0.2 to 2.5 % (w/v).

The fat emulsion of this invention can be prepared by a conventional method, for instance, in the following manner. A fixed amount of the compound [I] is dissolved in the above fat emulsion base at an appropriate temperature and thereto are added a fixed amount of the emulsifying agent and optionally other additives such as an isotonic agent and stabilizing agent, and the mixture is stirred under heating to obtain a uniform mixture, and thereto is added water. The mixture is treated with a homogenizer to give a crude oil-in-water type emulsion and then further homogenized with a pressure homogenizer.

The above emulsifying procedure is preferably carried out until the particles of dispersed oil and fat have a mean particle size of not more than 1 μm, more preferaly not more than 0.2 μm. Besides, the active compound [I] is usually contained in a concentration of 0.01 to 20 % (w/v), preferably 0.5 to 10 % (w/v), more preferably 1 to 3 % (w/v), based on whole weight of the composition.

The fat emulsion thus obtained may optionally be lyophilized. The lyophilized powdery product can easily be recovered to the original fat emulsion by re-dissolving it

in water, and hence, this invention includes also such a type of preparation as lyophilized product.

The fat emulsion of this invention can be administered to the patients by injection. The dose of the product may vary depending on the age and weight of the patient, the severity of the diseases, administration routes, and the like, but is usually in the range of 50 to 500 mg (converted as the amount of the active compound [I]) per one time in adult and it is administered one to three times per day in the above dose per one time.

The fat emulsion of this invention is useful for the treatment of various infectious diseases induced by various fungi, such as Trichophyton, Candida, Aspergillus, Cryptococcus, Coccidioides, etc., specifically such mycotic infections as trichophytosis, hemato-mycosis, penumonomycosis, peptic mycosis, urinary mycosis, mycotic meningitis, and the like.

This invention is illustrated by the following Examples and Experiments but should not be construed to be limited thereto.

Example 1

The compound [I] (4 g) is added to and dissolved in soybean oil (Japan Pharmacopeia) (20 g) with heating. To the mixture are added purified soybean phospholipid (3.6 g) and glycerin (5 g), and the mixture is vigorously stirred with heating, and to the resulting solution is added a

prescribed amount of distilled water. The mixture is stirred with Polytrone homogenizer to obtain a crude emulsion. The crude emulsion is further emulsified under pressure with Mantone-Gaurine homogenizer, and to the resulting mixture is added distilled water so as to make totally 200 ml, and the mixture is further homogenized to give an intravenously injectable fat emulsion containing the compound [I] wherein very fine particles of fats are dispersed. The dispersed fat particles have a mean particle size of 0.22 μm, and no particles having a size of more than 1 μm are contained in the fat emulsion.

Example 2

In the same manner as described in Example 1 by using soybean oil (Japan Pharmacopeia) (20 g), the compound [I] (2 g), purified soybean phospholipid (2.4 g), glycerin (5 g) and distilled water, there is obtained the desired fat emulsion (200 ml).

The dispersed fat particles in the product have a mean particle size of 0.11 μm, and no particles having a size of more than 1 μm are contained in the product.

Example 3

In the same manner as described in Example 1 by using soybean oil (Japan Pharmacopeia) (10 g), the compound [I] (1 g), purified soybean phospholipid (2.4 g), glycerin (5 g) and distilled water, there is obtained the desired fat emulsion (200 ml).

The dispersed fat particles in the product have a mean particle size of 0.14 μm, and no particles having a size of more than 1 μm are contained in the product.

Example 4

In the same manner as described in Example 1 by using soybean oil (Japan Pharmacopeia) (20 g), the compound [I] (4 g), purified soybean phospholipid (3 g), glycerin (5 g) and distilled water, there is obtained the desired fat emulsion (200 ml).

The dispersed fat particles in the product have a mean particle size of 0.22 μm, and no particles having a size of more than 1 μm are contained in the product.

Example 5

In the same manner as described in Example 1 by using Panacate® 810 (a mixture of triglycerides of medium fatty acids, manufactured by Nippon Oil and Fats Co., Ltd.) (20 g), the compound [I] (4 g), purified soybean phospholipid (3 g), glycerin (5 g) and distilled water, there is obtained the desired fat emulsion (200 ml).

The dispersed fat particles in the product have a mean particle size of 0.11 μm, and no particles having a size of more than 1 μm are contained in the product.

Example 6

The compound [I] (2 g) is added to and dissolved in soybean oil (Japan Pharmacopeia) (20 g) with heating. To the mixture are added purified soybean phospholipid (2.4 g), cholesterol (0.6 g) and glycerin (5 g), and the mixture is

vigorously stirred with heating, and to the resulting solution is added a prescribed amount of distilled water. The mixture is stirred under pressure with Polytrone homogenizer to obtain a crude emulsion. The crude emulsion is further emulsified under pressure with Mantone-Gaurine homogenizer, and to the resulting mixture is added distilled water so as to make totally 200 ml, and the mixture is further homogenized to give the desired fat emulsion. The dispersed fat particles in the product have a mean particle size of 0.14 μm, and no particles having a size of more than 1 μm are contained in the product.

Example 7

In the same manner as described in Example 1 by using soybean oil (Japan Pharmacopeia) (20 g), the compound [I] (4 g), purified soybean phospholipid (1.8 g), tween 80 (1.8 g), glycerin (5 g) and distilled water, there is obtained the desired fat emulsion (200 ml).

The dispersed fat particles in the product have a mean particle size of 0.11 μm, and no particles having a size of more than 1 μm are contained in the product.

Example 8

To the fat emulsion obtained in Example 1 is added albumin (5 g), and the mixture is lyophilized to give a powder of the fat emulsion containing the compound [I].

Experiments of activities:

The excellent antifungal activities and safety of the fat emulsions of this invention were investigated by the

following experiments.

Experiment 1

Activity against Candia albicans:

(a) Test animals:

Male CRJ:ICR mice (5 week age, 10 mice per group) were used.

(b) Injection products to be tested:

The fat emulsion obtained in Example 1; an injection solution (HCO 60 preparation) wherein the compound [I] was dissolved in a concentration of 2 % (w/v) in an aqueous solution of 10 % (w/v) of polyoxyethylene hydrogenated castor oil and 5 % (w/v) of glucose; and a solvent for the HCO 60 preparation (i.e. an aqueous solution of 10 % (w/v) of polyoxyethylene hydrogenated castor oil and 5 % (w/v) of glucose (as a control)

(c) Method:

The test mice was infected with Candida albicans (clinically isolated strain) by injecting the microorganism in an amount of 5 x $10^6$ cells/mouse through the tail vein. One hour after the infection, the test injection products were each injected through the tail vein, and the survival days of mice were measured.

(d) Experimental results:

The evaluation of effect was calculated by the following formula:

$$T/C = \frac{\text{Average survival days in each test group}}{\text{Average survival days in control group}} \times 100$$

The results are shown in Table 1. As is clear from the results, the compound [I] showed remarkedly excellent effect against the infection.

Table 1

| Injection products to be tested | Dose of compound [I] (mg/kg) | T/C (%) |
|---|---|---|
| HCO 60 preparation | 60 | 262 |
| Product of Ex. 1 | 60 | 289 |
| Control solution | 0 | 100 |

Experiment 2

Irritation to blood vessel:

(a) Injection products to be tested:

The fat emulsions obtained in Examples 2 and 3; and injection solutions (HCO 60 preparations) wherein the compound [I] was dissolved in a concentration of 0.1, 0.2, 0.5, or 0.6 % (w/v) in an aqueous solution of 10 % (w/v) of polyoxyethylene hydrogenated castor oil and 5 % (w/v) of glucose.

(b) Method:

To SD rats (6 week age) was injected via tail vein the above injection products to be tested once a day every day until anthraconecrosis of the injection region appeared and thereby the injection could be no more done, and the number of total days was counted.

(c) Experimental results:

The results are shown in Table 2. As is clear from

the results, the fat emulsions of this invention showed little irritation to the blood vessel.

Table 2

| Injection products to be tested | Compound [I] | | Total days until no more injection could be done |
|---|---|---|---|
| | Concn. [% (w/v)] | Dose (mg/kg) | |
| HCO 60 preparations | 0.1<br>0.2<br>0.5<br>0.6 | 10<br>10<br>25<br>60 | 14<br>7<br>7<br>1 |
| Product of Ex. 3 | 0.5 | 25 | More than 14 |
| Product of Ex. 2 | 1.0 | 50 | More than 14 |

Experiment 3

Acute toxicity:

(a) Test animals:

Male DDY mice (weighing 20 - 25 g) were used in 10 mice per group.

(b) Injection products to be tested:

The fat emulsion obtained in Example 1; and an injection solution (HCO 60 preparation) wherein the compound [I] was dissolved in a concentration of 2 % (w/v) in an aqueous solution of 10 % (w/v) of polyoxyethylene hydrogenated castor oil and 5 % (w/v) of glucose.

(c) Method:

The injection products to be tested were each injected via tail vein of the mice, and the animals were observed for 14 days.

(d) Experimental results:

The results are shown in Table 3. As is clear from the results, the fat emulsion of this invention showed clearly lower toxicity in comparison with the HCO 60 preparation and hence were very safe.

Table 3

| Injection products to be tested | $LD_{50}$ (mg/kg) |
|---|---|
| HCO 60 preparation | 103 |
| Product of Ex. 1 | 200 |

As is clear from the above, when the compound [I] is formed into the fat emulsion as in the present invention, it can be administered with higher safety without lowering of the excellent antifungal activities and hence can be used as an antifungal agent for various mycotic infections, particularly for deep mycotic infections, effectively and safely.

What is claimed is:

1. An oil-in-water type fat emulsion, which comprises

(a) 0.01 to 20 % (w/v) of an active compound of the formula:

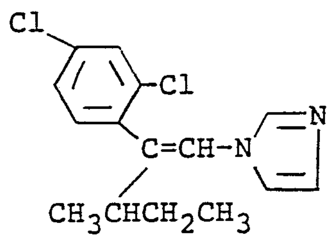

[I]

(b) 5 to 50 % (w/v) of at least one fat emulsion base selected from the group consisting of vegetable oils, triglycerides of a fatty acid having 8 to 12 carbon atoms, and di- and mono-glycerides of a fatty acid having 6 to 18 carbon atoms,

(c) 0.05 to 5 % (w/v) of at least one emulsifying agent selected from the group consisting of a phospholipid having a content of phosphatidylcholine of 85 % (w/w) or more and a nonionic surfactant, and

(d) remainder of water.

2. The oil-in-water type fat emulsion according to claim 1, wherein (a) the active compound [I], (b) the fat emulsion base, and (c) the emulsifying agent are incorporated in an amount of 0.5 to 10 % (w/v), 5 to 20 % (w/v), and 0.5 to 3 % (w/v), respectively.

3. The oil-in-water type fat emulsion according to claim 1, wherein an isotonic agent selected from the group consisting of glycerin, sugar alcohols, monosaccharides,

disaccharides and amino acids is further incorporated in an amount of 1 to 5 % (w/v).

4. The oil-in-water type fat emulsion according to claim 1, wherein a stabilizing agent selected from cholesterol in an amount of not more than 1.2 % (w/v) and tocopherol in an amount of not more than 2.5 % (w/v) is further incorporated.

5. The oil-in-water type fat emulsion according to claim 3, wherein a stabilizing agent selected from cholesterol in an amount of not more than 1.2 % (w/v) and tocopherol in an amount of not more than 2.5 % (w/v) is further incorporated.

6. The oil-in-water type fat emulsion according to claim 1, wherein albumin is further incorporated as a stabilizing agent in an amount of 0.2 to 2.5 % (w/v).

7. A process for preparing an oil-in-water type fat emulsion comprising

(a) 0.01 to 20 % (w/v) of an active compound of the formula:

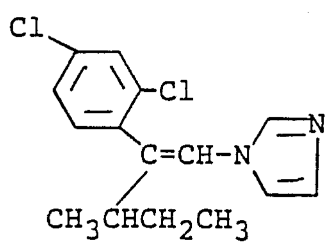

[I]

(b) 5 to 50 % (w/v) of at least one fat emulsion base selected from the group consisting of vegetable oils, triglycerides of a fatty acid having 8 to 12 carbon atoms, and di- and mono-glycerides of a fatty acid having 6 to 18

carbon atoms,

(c) 0.05 to 5 % (w/v) of at least one emulsifying agent selected from the group consisting of a phospholipid having a content of phosphatidylcholine of 85 % (w/w) or more and a nonionic surfactant, and

(d) remainder of water,

which comprises dissolving the fixed amount of the active compound (a) in the fixed amount of the fat emulsion base (b), adding thereto a fixed amount of the emulsifying agent (c) and optionally an isotonic agent and a stabilizing agent, stirring the mixture to form a uniform mixture, adding thereto water, and then homogenizing the mixture.

European Patent Office

# EUROPEAN SEARCH REPORT

0 257 454

Application Number

EP 87 11 1680

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | EP-A-0 049 913 (GIST-BROCADES N.V.)<br>* Claim 1; page 51, lines 1-5,18-20 * | 1-7 | A 61 K 9/10<br>A 61 K 47/00<br>A 61 K 31/415 |
| D,Y | EP-A-0 171 084 (LEDERLE (JAPAN) LTD)<br>* Claims 3-14,17,21; page 36, examples 1-3 * | 1-7 | |
| A | EP-A-0 100 448 (A. NATTERMANN & CIE GmbH)<br>* Claims 5,8; page 9, example 2 * | 1-7 | |
| A | EP-A-0 143 305 (BAYER AG)<br>* Page 9; pages 18-19, examples 5-7; abstract * | 1-7 | |

TECHNICAL FIELDS SEARCHED (Int. Cl.4)

C 07 D
A 61 K

The present search report has been drawn up for all claims

| Place of search | Date of completion of the search | Examiner |
|---|---|---|
| THE HAGUE | 25-11-1987 | FOERSTER W.K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)